# EUROPEAN PATENT SPECIFICATION

(11) **EP 4 328 919 B1**
(45) Date of publication and mention of the grant of the patent: **22.07.2026**
(21) Application number: 23171753.9
(22) Date of filing: 05.05.2023
(51) Int. Cl.: G16B 35/00, G16B 20/30, G16B 30/00, G16B 40/20, A61K 39/00

(54) **IMMUNOGENICITY PREDICTION DEVICE, IMMUNOGENICITY PREDICTION METHOD AND COMPUTER PROGRAM FOR SYNTHETIC LONG PEPTIDE DESIGN**
IMMUNOGENITÄTSVORHERSAGEVORRICHTUNG, IMMUNOGENITÄTSVORHERSAGEVERFAHREN UND COMPUTERPROGRAMM FÜR SYNTHETISCHES LANGES PEPTIDDESIGN
DISPOSITIF DE PRÉDICTION D'IMMUNOGÉNICITÉ, PROCÉDÉ DE PRÉDICTION D'IMMUNOGÉNICITÉ ET PROGRAMME INFORMATIQUE POUR LA CONCEPTION DE PEPTIDES LONGS SYNTHÉTIQUES

(30) Priority: 23.08.2022 KR 20220105655
(43) Date of publication of application: 28.02.2024
(73) Proprietor: Theragen Bio Co., Ltd., Gyeonggi-do 13488 (KR)
(72) Inventor: HWANG, Tae soon, Seongnam-si (KR); PAIK, Soon Myung, Seongnam-si (KR); HONG, Seong Eui, Seongnam-si (KR); KIM, Hae Suk, Seongnam-si (KR); NOH, Eon Ji, Seongnam-si (KR); JUNG, Min Ho, Seongnam-si (KR); KIM, Seong Gwang, Seongnam-si (KR); KIM, In Young, Seongnam-si (KR)
(74) Representative: M. Zardi & Co S.A.

(56) References cited:
- JINGCHENG WU ET AL: "DeepHLApan: A Deep Learning Approach for Neoantigen Prediction Considering Both HLA-Peptide Binding and Immunogenicity", FRONTIERS IN IMMUNOLOGY, vol. 10, 1 November 2019 (2019-11-01), pages 2559, XP055682961, DOI: 10.3389/fimmu.2019.02559
- CHEN BINBIN ET AL: "Predicting HLA class II antigen presentation through integrated deep learning", NATURE BIOTECHNOLOGY, NATURE PUBLISHING GROUP US, NEW YORK, vol. 37, no. 11, 14 October 2019 (2019-10-14), pages 1332 - 1343, XP036920809, ISSN: 1087-0156, [retrieved on 20191014], DOI: 10.1038/S41587-019-0280-2
- XIAOYUN YANG ET AL: "DeepNetBim: deep learning model for predicting HLA-epitope interactions based on network analysis by harnessing binding and immunogenicity information", BMC BIOINFORMATICS, BIOMED CENTRAL LTD, LONDON, UK, vol. 22, no. 1, 5 May 2021 (2021-05-05), pages 1 - 16, XP021290448, DOI: 10.1186/S12859-021-04155-Y
- CHEN XIAOTONG ET AL: "Personalized neoantigen vaccination with synthetic long peptides: recent advances and future perspectives", vol. 10, no. 13, 15 May 2020 (2020-05-15), AU, pages 6011 - 6023, XP093117119, ISSN: 1838-7640, Retrieved from the Internet <URL:https://www.thno.org/v10p6011.pdf> [retrieved on 20240109], DOI: 10.7150/thno.38742

## Description

### [TECHNICAL FIELD]

Embodiments of the present disclosure relate to an immunogenicity prediction device, an immunogenicity prediction method and a computer program for designing synthetic long peptides (SLPs) used in cancer vaccines. More specifically, the embodiments are characterized by calculating scores for the immunogenicity of synthetic long peptides injected as cancer vaccines by using models that predict the HLA binding affinity, T cell recognition ability, and peptide cleavage.

### [BACKGROUND ART]

Cancer is one of the most common causes of death worldwide. About 10 million new cancer cases occur each year, and cancer accounts for about 12% of all death causes, which is the third leading cause of death. Our immune system does not attack normal cells because self-recognizing T cells are eliminated through thymic selection. This phenomenon is called central tolerance. Cancer is a disease caused by the accumulation of mutations in the genome. Solid cancer cells such as gastric cancer, colon cancer, and breast cancer have an average of about 60 non-synonymous mutations, while lung cancer or melanoma caused by carcinogens has more than 150 non-synonymous mutations.

When a mutation occurs, the cancer cells produce an abnormal protein. In this case, since the immune system has never seen the mutant protein during thymic selection process, central tolerance does not apply. Therefore, it may be seen that all mutant proteins have potential immunogenicity. However, most mutations involve intracellular proteins, wherein these proteins cannot be located at the surface of tumor cells, and thus cannot be directly presented to immune cells. Mutations in cancer cells can only be recognized by the immune system through a process of presenting short peptide fragments, called epitopes, on the surface of cancer cells. These epitopes are typically 8 to 10 amino acids in length and are presented on the cell surface as a complex with the class 1 major histocompatibility complex (MHC) protein, also known as Human Leukocyte Antigen (HLA) in humans. At this time, the mutant peptide bound to class 1 MHC and presented on the surface of tumor cells is called a neoepitope or a neoantigen, and the neoepitope and MHC complex is called pMHC, (or pHLA in humans). Such neoepitope is a marker that helps CD8 cytotoxic T cells to recognize cancer cells as an external attacker.

However, for an immune response to be activated against mutations present in cancer cells, the cancer cell-derived neoantigen pMHC must be presented to T lymphocytes along with co-stimulatory molecules by antigen presenting cells (APCs), particularly dendritic cells. Unlike other somatic cells, APCs also present longer peptides as neoantigens as a complex with class 2 MHC protein, activating CD4 T helper cells. In order to be primed against neoantigens, CD8 T-cells has to make immunological synapse with dendritic cells cross presenting class I pMHC together with co-stimulatory signals and also need help from CD4 helper T cells activated by class II pMHC. Although these activated T immune cells attempt to attack cancer cells, the cancer cell can exhibit an immune checkpoint that paralyzes the activated T cells, inducing immunological exhaustion. The majority of immune therapies currently in clinical use are immune checkpoint inhibitors that act through reactivation of the exhausted effector T-cells. Immune checkpoint inhibitors have clinical efficacy only in patients who once had robust immune response against tumor cells, which are now dysfunctional. Recently, it has been shown that many neoantigens fail to prime cytotoxic T-cells, i.e., ignored by immune system, because the number of pMHC on the surface of lymph node resident dendritic cells simply do not reach the priming threshold. Neoantigen vaccination has been shown to overcome immunological ignorance and result in de novo priming of naive T-cells.

In other words, neoantigen-targeting cancer vaccines are expected to be applicable to carcinomas that do not respond to existing immune checkpoint inhibitors. Even in the target patient group to which immune checkpoint inhibitors are indicated, a greater therapeutic effect can be expected with combination therapy with neoantigen targeted vaccine.

Previous cancer vaccines served 'tumor-associated antigens' as a therapeutic target, which refers to a 'normal' protein that shows an abnormally higher expression level in cancer cells than in normal cells. The targets that commonly appear in this way can be used to create off-the-shelf vaccines, but there is a drawback of low antigenicity due to central tolerance, and no vaccine has yet proven to be clinically effective. Unlike such 'tumor-associated antigens', neoantigens are not subject to central tolerance, and thus, can be an ideal cancer vaccine target.

Meanwhile, HLA genes that encode MHC proteins have the highest polymorphism with more than 13,000 alleles. Polymorphism cause differences in the peptide binding groove sequence of MHC proteins, whereby MHC proteins have various affinities to peptides. Therefore, even peptides in which the same mutation has occurred can be presented as neoantigens only in patients with specific HLA types with high affinity. Because of this MHC restriction, only less than 10% of the mutated peptides are presented by MHC, and neoantigen repertoire are unique to each cancer patient, and for this reason, cancer vaccines must be custom designed for each patient.

Cancer vaccines can be designed in the form of synthetic long peptides (SLPs) containing mutations, mRNA or DNA encoding them, or antigen presenting cells such as dendritic cells loaded with them. Through many studies so far, it has been found that in the case of a neoantigen peptide vaccine containing a minimum epitope with a length of 8-10mer, antigens are presented not only by dendritic cells, which are specialized antigen-presenting cells, but also by unspecialized antigen-presenting cells, thus not effectively activating the immune response, whereas the 25-mer long SLP containing the neoantigen is phagocytosed by dendritic cells, a portion of which is degraded into 8-10 mer peptides, and then cross-presented as class 1 pMHC with auxiliary proteins, and a portion is cross-presented as class 2 pMHC to activate the immune response more effectively.

So far, in all preclinical and clinical trials, vaccines have used SLPs, or tandem minigene mRNA/DNA encoding SLP(s), covering the top 20 mutations that are likely to be neoantigens. The structure of the SLP or SLP mRNA/DNA typically involves placing the mutations that generated the neoantigens in the middle. In the case of peptide vaccines, only when it is difficult to synthesize a 25mer SLP in which a mutation is positioned at the center, the position of the mutation is inevitably changed or the length is shortened. In the case of mRNA or DNA vaccines, the mutation is always positioned at the center.

However, no scientific or experimental rationale for positioning the mutation at the center of the SLP has been presented. Previous studies have never posed the question of the effect of the position of mutations in the SLP/mRNA/DNA sequence on immunogenicity.

Since effective priming requires cross presentation of both class I and class II pMHCs, we reasoned that the examination of the position of class I and II core epitopes within the 25mer epitopes catalogued in the Immune Epitope Database (IEDB), which records extensive information on antigen-HLA binding and immunogenicity, may provide insight as to the ideal design of the SLP to ensure presentation of both classes of pMHCs. When the unique arrangement of these antigens was learned through machine learning and scored accordingly, it was proven that higher scores corresponded to higher immunogenicity.

In order for the neoantigen vaccine to be effective, potentially immunogenic neoantigen candidates have to be selected, and SLPs incorporating them have to be designed to maximize the chance of naive T-cell priming. The present disclosure presents a computational algorithm that can help design a SLP with a maximum probability of cross-presentation by both HLA classes I and II pMHCs from a given mutated core epitope, thus maximizing the immunogenicity of the resulting SLP.

The above-mentioned background art is technical information that the inventor possessed for derivation of the invention or acquired during the derivation process of the invention, which cannot necessarily be said to be known art disclosed to the general public prior to filing the present disclosure.

The publication Frontiers in Immunology, vol.10, 2019, page 2559 discloses an immunogenicity prediction method using deep learning techniques to predict neoantigens considering both the possibility of HLA-peptide binding (binding model) and the potential immunogenicity (immunogenicity model). The method includes the steps of acquiring information about synthetic long peptides for treating carcinoma; processing the obtained data to output one or more antigen feature values; inputting a neoantigen peptide sequence (HLA pseudo sequence) and outputting one or more neoantigen feature values; outputting an immunogenicity score based on the antigen- and neoantigen feature values previously determined.

This publication does not disclose any one of: generating a vector of cleavage probability based on the sequence information of the synthetic long peptides; inputting the cleavage probability vector for each position of the synthetic long peptides through an immunogenicity prediction device to output one or more cleavage feature values; outputting an immunogenicity score of a synthetic long peptide using a model trained with cleavage feature values.

### [SUMMARY OF THE INVENTION]

The invention is set out in the appended claims.

### [DETAILED

### DESCRIPTION OF THE INVENTION]

### [Technical Problem]

Embodiments disclosed herein aim at providing an immunogenicity prediction device, an immunogenicity prediction method and a computer program for synthetic long peptides, that calculates scores for the immunogenicity of synthetic long peptides injected as cancer vaccines by using models that predict cleavage prediction information for synthetic long peptides applied to target cells of patients, HLA binding affinity of neoantigens in synthetic long peptides, T cell recognition ability, etc.

### [Technical Solution]

According to embodiments of the present disclosure, there is provided an immunogenicity prediction method according to the independent claim 1. The method comprises inter alia the steps of: acquiring information about synthetic long peptides for treating carcinoma in a subject through an immunogenicity prediction device; processing data obtained from the information about synthetic long peptides by one processing method of embedding, one-hot encoding, and BLOSUM through the immunogenicity prediction device, and outputting one or more antigen feature values based on the processed data; inputting the cleavage probability vector for each position of the synthetic long peptides through the immunogenicity prediction device to output one or more cleavage feature values; inputting neoantigen peptide sequences corresponding to an HLA class I sequence, an HLA class II sequence within the synthetic long peptides through the immunogenicity prediction device to output one or more neoantigen feature values related to the immunity and binding affinity present in the synthetic long peptide; and outputting an immunogenicity score of the neoantigen peptide through the immunogenicity prediction device in consideration of the one or more antigen feature values, the one or more cleavage feature values, and the one or more neoantigen feature values.

The step of outputting the one or more antigen feature values may comprise outputting a first antigen feature value using a model learned from data obtained by embedding the sequence information of the synthetic long peptide, outputting a second antigen feature value using a model learned from data obtained by one-hot encoding the sequence information of the synthetic long peptide, and outputting a third antigen feature value using a model learned from data obtained by BLOSUM processing the sequence information of the synthetic long peptide.

The cleavage probability vector for each position of the synthetic long peptide may be a cleavage probability vector for each position, when a synthetic long peptide is cleaved with proteosomes or cathepsins, which are cleavage enzymes present in the subject.

The step of outputting neoantigen feature values related to the immunity and binding affinity present in the synthetic long peptide may comprise outputting a first neoantigen feature value related to the immunity using a T cell activity data and a model learned with immunity to neoantigen peptides, outputting a second neoantigen feature value corresponding to the binding affinity between the neoantigen and HLA class I or II using a model learned by inputting the binding data for complexes of neoantigens present in synthetic long peptides and HLA class I or HLA class II alpha and beta alleles, and outputting a third neoantigen feature value that is a product of the first neoantigen feature value and the second neoantigen feature value.

The synthetic long peptide may have a length of 40mers or less.

The synthetic long peptide may be formed such that a neoantigen or epitope sequence is positioned at the center.

According to the embodiments disclosed herein, for synthetic long peptides containing multiple neoantigen peptides, a method for determining the synthetic long peptide with the highest immunogenicity score may include repeating steps of outputting one or more antigen characteristic values, outputting one or more cleavage characteristic values, outputting one or more neoantigen characteristic values, and outputting the immunogenicity score, and determining the synthetic long peptide with the highest immunogenicity score.

According to the embodiments disclosed herein, there is provided an immunogenicity prediction device according to the independent claim 9. The device comprises inter alia: a data input unit that acquires information about synthetic long peptides for treating carcinoma in a subject; an antigen feature output unit that processes data obtained from the information about the synthetic long peptide by one processing method of embedding, one-hot encoding, and BLOSUM, and outputs one or more antigen feature values based on the processed data; a cleavage feature output unit that inputs the cleavage probability vector for each position of the synthetic long peptide to output one or more cleavage feature values; a unit that outputs one or more feature values related to the immunity and binding affinity present in a synthetic long peptide by inputting a neoantigen peptide sequence corresponding to an HLA class I sequence and an HLA class II sequence present in a synthetic long peptide; and an immunogenicity score output unit that outputs an immunogenicity score of the synthetic long peptide in consideration of the one or more antigen feature values, the one or more cleavage feature values, and the one or more neoantigen feature values.

According to the embodiments disclosed herein, there is provided a computer program stored on a computer-readable storage medium to execute any one of the methods according to the embodiments of the present disclosure using a computer.

In addition, other methods and systems for implementing the present disclosure, and computer readable recording media recording a computer program for executing the methods are further provided.

Other aspects, features and advantages other than those described above will become apparent from the following drawings, claims and detailed description of the invention.

### [Advantageous Effects]

According to any one of the above-mentioned technical solutions, it is possible to calculate scores for the immunogenicity of synthetic long peptides (SLPs) injected as cancer vaccines using models that predict HLA binding affinity, T cell recognition, and cleavage prediction information of neoantigens within the synthetic long peptides applied to the target cells of patients.

### [BRIEF DESCRIPTION OF THE DRAWINGS]

FIG. 1 is a block diagram of an immunogenicity prediction device 100 according to embodiments of the present disclosure.
FIG. 2 is a flowchart of an immunogenicity prediction method according to embodiments of the present disclosure.
FIG. 3 is an illustrative diagram of a model that outputs immunogenicity scores for synthetic long peptides.
FIG. 4a is an illustrative diagram of a BLOSUM matrix used in the immunogenicity prediction device 100.
FIG. 4b is an illustrative diagram which is encoded by one-hot encoding V1 and product V2 between one-hot encoding and a BLOSUM matrix for a synthetic long peptide sequence based on the BLOSUM matrix of FIG. 4a.
FIG. 5a is an illustrative diagram of the relative positions of neoantigen peptides binding to HLA classes I and II within long peptides known to bind to the existing HLA class I or II.
FIG. 5b is a diagram which predicts the position of neoantigen peptides binding to HLA class II on the right side of the synthetic long peptide in which the neoantigen binding to HLA class I is arranged at the center.
FIG. 5c is a diagram which predicts the position of neoantigen peptide binding to HLA class II on the left side of the synthetic long peptide in which the neoantigen binding to HLA class I is arranged at the center.
FIG. 5d is a diagram which assumes the position of the mutation within the neoantigen binding to HLA class I from the first to the fifth, and then predicts the position of the neoantigen binding to HLA class II, when a mutation occurs at the center of the synthetic long peptide.
FIG. 6 is a diagram showing the results of inhibiting cancer growth with neoantigen peptides found by the immunogenicity prediction device.
FIG. 7 is an illustrative diagram of a graph of changes in the size of skin cancer in B16F10 melanoma mice.
FIG. 8 is a diagram that presents the immunogenicity prediction score for the existing method for five synthetic long peptides (SLPs) injected into B16F10 melanoma mice, i.e., a method considering cross-presentation relative to a method in which mutation is arranged at the center (Reference), i.e., a method in which the neoantigen corresponding to HLA class I is arranged at the center (Target).
FIG. 9 is an illustrative diagram of vectors input to the immunogenicity prediction device 100.
FIG. 10 is an illustrative diagram of the results of predicting the immunogenicity of synthetic long peptides related to frequently observed mutations (Recurrent variant) and only once observed mutation (Non-recurrent variant) within the cancer database TCGA (The Cancer Genome Atlas).

### [DETAILED DESCRIPTION OF THE EMBODIMENTS]

Hereinafter, the constitution and operation of the present disclosure will be described in detail with reference to embodiments of the present disclosure shown in the accompanying drawings.

The invention can have various embodiments. Particular embodiments will be illustrated in the drawings and described in detail in the written description. Advantageous effects and features of the present disclosure, and methods for achieving them will become apparent with reference to the embodiments described later in detail together with the drawings. However, this invention is not limited to the embodiments disclosed below and can be embodied in various forms.

Hereinafter, one or more embodiments of the present disclosure will be described below in more detail with reference to the accompanying drawings. Those components that are the same or are in correspondence are rendered the same reference numeral, and redundant explanations are omitted.

Further, the term "training" or "learning" as used herein refers to performing a machine learning through computing according to a procedure and it will be apparent to those skilled in the art that the term is not intended to refer to a mental action such as an educational activity of a human.

In the following embodiments, the terms "first," "second," etc. are not meant to be limiting, and these terms are only used to distinguish one constitutional element from the other constitutional elements.

In the following embodiments, the singular forms "a," "an" and "the" are intended to include plural forms, unless the context clearly indicates otherwise.

In the following embodiments, the terms "comprise," "include", "have", etc. are used herein to specify the presence of stated features, or components, but do not preclude the presence or addition of one or more other features, or components.

Sizes of elements in the drawings may be exaggerated or reduced for convenience of explanation. In other words, since sizes and thicknesses of components in the drawings are arbitrarily illustrated for convenience of explanation, the present disclosure is not limited thereto.

When a certain embodiment may be implemented differently, a specific process order may be performed differently from the described order. For example, two consecutively described processes may be performed substantially at the same time or performed in an order opposite to the described order.

A neoantigen refers to a peptide that is generated through mutation, abnormal fusion between genes, aberrant alternative splicing, and the like, and causes an immune response. That is, the neoantigen may be an immunogenic peptide. Because the neoantigen is selectively expressed only in the patient's tumor, it can induce a tumor-specific T cell response. The neoantigen can be induced by tumor cell-specific mutations and can be expressed as epitopes on tumor cells. Hereinafter, for simplicity of explanation, the immunogenic peptides are named and described as neoantigen peptides

As used herein, the synthetic long peptide (SLP) included in the vaccine may have a length of less than 40 mer, and may be degraded into a plurality of peptides. The degraded peptides may be bound to HLA class I and/or HLA class II, and the like. The degraded peptides can bound to HLA class I and/or HLA class II and the like, and then trigger an immune response by T cells.

Here, the peptide may refer to a peptide or polypeptide composed of amino acid sequences. An immune system exists in living organisms to remove foreign materials that are not derived from genetic information within each species, and particularly, there exist immunogenic peptides that can induce an immune response among exogenously-derived peptides. Mutations that occur differently from the original genetic information during cancer development also generate these immunogenic peptides, so that such peptides can bind to HLA class I or II through a series of processes in the immune system. Furthermore, the immunogenic peptide can have a mutated amino acid sequence, and its amino acid length may be 25 or less, but is not limited thereto and may be of various lengths.

Here, the T cell activity data is the data obtained by measuring the immune response generated when stimulated by the binding of a specific peptide sequence to a specific HLA class I or II allele, and is acquired from data such as intracellular cytokine expression values and immune cell-specific activation marker expression values detected by immunogenicity measurement experimental methodologies including multimer/tetramer and ELISPOT.

Here, the neoantigen peptide may be 40 mer or less. The synthetic long peptide may be formed such that a neoantigen sequence binding to HLA class I is positioned at the center.

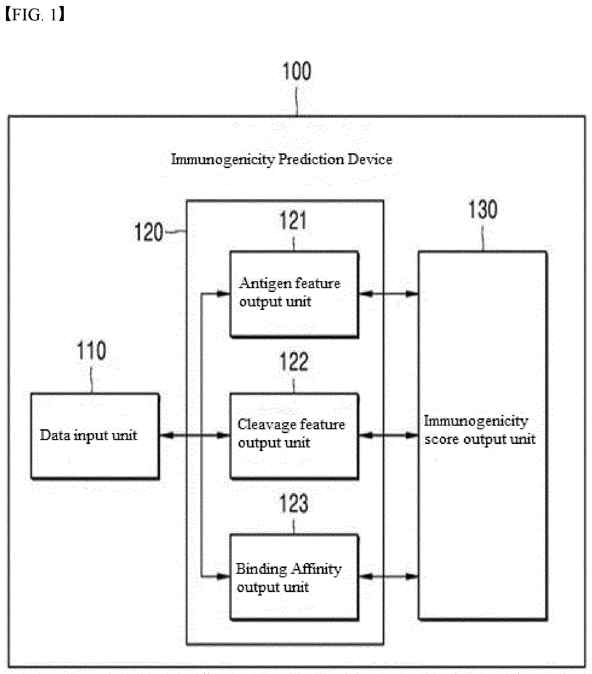

FIG. 1 is a block diagram of an immunogenicity prediction device 100 according to embodiments of the present disclosure.

The data input unit 110 may input the sequence of a synthetic long peptide as input data. For model learning, the sequence of a long peptide having the same length as the synthetic long peptide injected into a patient, especially a long peptide containing neoantigens that bind to HLA classes I and II among the peptides produced by degrading long peptides can be input as input data.

The data input unit 110 searches for synthetic long peptide candidates applicable to the patient in consideration of the patient's cancer disease, and can generate input data for such synthetic long peptide candidates.

The data input unit 110 may acquire long peptides that activates CD8+ T cells and CD4+ T cells based on Immune Epitope Database data, that is, acquire long peptides having the same length as synthetic long peptides used for cancer vaccines, among peptides that bind to HLA classes I and II. The CD8+ T cell activity collects HLA class I and epitopes for which binding information exists, and the CD4+ T cell activity can collect HLA class II and epitopes for which binding information exists. Here, such a long peptide having immunogenicity may have a unique pattern in which amino acids of Acidic-Aromatic-Basic are sequentially arranged.

The antigen feature extraction unit 121 can process the sequence information of the neoantigen peptide by at least one processing method of embedding, one-hot encoding, and BLOSUM processing, and extract an antigen feature value from the processed data. The antigen feature extraction unit 121 includes a model learned from data obtained by embedding sequence information, a model learned from data obtained by one-hot encoding sequence information, and a model learned from data obtained by BLOSUM processing sequence information, so that data obtained by processing sequence information by each method can be input in one of the above models to output antigen feature values.

The embedding method may be to convert amino acid sequences contained in the peptide sequence into corresponding values. At this time, it can be experimentally and empirically compared with all amino acid sequences contained in the peptide sequence, and converted into corresponding values.

The one-hot encoding method may be to convert one or more amino acid sequences contained in the peptide sequence into 0 and 1 at each position. One-hot encoding can be performed by setting to 1 at positions where each amino acid sequence included in the peptide sequence exists, and setting to 0 at positions where each amino acid sequence does not exist.

The BLOSUM processing method can refer to computing the product between the one-hot encoding matrix and the BLOSUM matrix.

The antigen feature extraction unit 121 can input the sequence information of the synthetic long peptide to the model learned from the embedded data to output a first antigen feature value, input the sequence information of the neoantigen peptide to a model learned from the one-hot encoded data to output a second antigen feature value, and input the sequence information of the synthetic long peptide to the model learned from the BLOSUM processed data to output the third antigen feature value.

At this time, the model learned from the embedded data may be a model that inputs the sequence information of the neoantigen peptide to the model learned from the one-hot encoded data to output the second antigen feature value. Antigen feature values of synthetic long peptides used to learn this model may utilize values stored in a database or values acquired through experiments.

The model learned from the one-hot encoded data may be a model that is learned by inputting data which is obtained by one-hot processing a plurality of synthetic long peptides, and outputting antigenic feature values of the synthetic long peptides. Antigen feature values of peptides used to learn this model may utilize values stored in a database or values acquired through experiments.

The model learned from BLOSUM-processed data may be a model that is learned by inputting data which is obtained by BLOSUM processing a plurality of synthetic long peptides, and outputting the antigenic feature values of the synthetic long peptides. Antigen feature values of synthetic long peptides used to learn this model may utilize values stored in a database or values acquired through experiments.

The cleavage feature extraction unit 122 may generate a vector of cleavage probability from the sequence information of synthetic long peptides. The cleavage feature prediction unit 122 may output a cleavage feature value using a cleavage prediction model learned by inputting a vector of cleavage probability.

Here, the cleavage prediction model may be a model that is learned from learning data of cleavage feature values through the cleavage probability vectors for each position of synthetic long peptides, and the synthetic long peptides. When synthetic long peptides are injected into the body of a patient, animal, etc., they are cleaved and degraded through predetermined materials present in the body. At this time, the material for cleaving the synthetic long peptides may include proteosome, cathepsin, and the like.

Synthetic long peptides can be cleaved into two or more peptides and bound to HLA class I and/or HLA class II in the body to operate. Some of the cleaved peptides bind to HLA class I, and some of the cleaved peptides can bind to HLA class II.

The binding affinity output unit 123 can input the neoantigen peptide sequence, HLA class I sequence, and HLA class II sequence present in the synthetic long peptide to output neoantigen feature values related to the immunity and binding affinity to the neoantigen peptide sequence. At this time, the binding affinity output unit 123 may output a first feature value related to the immunity using T cell activity data and a model learned by immunity to the peptides.

The binding affinity output unit 123 can input peptides present in synthetic long peptides and HLA class I or II allele sequences as the T cell activity data to output first neoantigen feature values corresponding to immunity to the peptides. The binding affinity output unit 123 may output first neoantigen feature values corresponding to immunity to peptides using a T cell activity data and a model learned with immunity to peptides.

The binding affinity output unit 123 can input binding data for peptides present in the synthetic long peptide and HLA class I or II, and output second neoantigen feature values corresponding to the binding affinity to peptides.

The binding affinity output unit 123 may output a third neoantigen feature value calculated by the product of the first neoantigen feature value and the second neoantigen feature value.

Synthetic long peptides can be cleaved and degraded in a subject and then bound to HLA class I and HLA class II to output a therapeutic effect. The degree to which the peptides present within synthetic long peptides bind to HLA class I or HLA class II can be predicted by a second neoantigen feature value related to the binding affinity. The degree to which the peptides present within the synthetic long peptide increase the immunity of the subject can be predicted by the first neoantigen characteristic value related to the immunity.

The immunogenicity score output unit 130 may output immunogenicity scores for peptides in consideration of the first to third antigen feature values, the cleavage feature value, and the first to third neoantigen feature values. The immunogenicity score for the peptides may be a value that is output by inputting first to third antigen feature values, cleavage feature values, and first to third neo-antigen feature values. At this time, the immunogenicity score for the peptides may be determined by a return value for a model learned from the first to third antigen feature values, the cleavage feature value, and the first to third neoantigen feature values. The model learned from the first to third antigen feature values, the cleavage feature value, and the first to third neoantigen feature values is learned by a machine learning method, and can be learned by methods such as supervised learning, unsupervised learning, and reinforced learning.

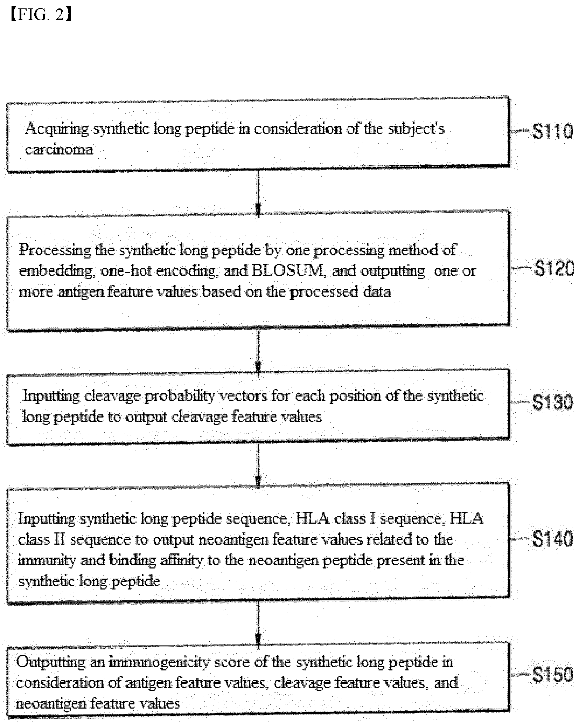

FIG. 2 is a flowchart of an immunogenicity prediction method according to embodiments of the present disclosure.

In S110, the immunogenicity prediction device 100 may acquire information about synthetic long peptides for treating carcinoma of a subject. The immunogenicity prediction device 100 may information about input synthetic long peptides, peptides generated by degradation of the synthetic long peptides, and the binding positions of the peptides as input data. Information about synthetic long peptides and peptides generated by degradation of the synthetic long peptides, and information about the binding position when the peptides are bound in the target cell, can be acquired from a model learned from an external database.

The immunogenicity prediction device 100 searches for synthetic long peptide candidates applicable to the patient in consideration of the patient's cancer disease, and can generate input data for candidates of these synthetic long peptides.

In S120, the immunogenicity prediction device 100 can process the sequence information of synthetic long peptides by at least one processing method of embedding, one-hot encoding, and BLOSUM processing, and extract antigen feature values from the processed data. The immunogenicity prediction device 100 includes a model learned from data obtained by embedding sequence information, a model learned from data obtained by one-hot encoding sequence information, and a model learned from data obtained by BLOSUM processing sequence information, so that data obtained by processing sequence information by each method can be input in one of the above models to output antigen feature values.

The embedding method may be to convert amino acid sequences contained in the peptide sequence into corresponding values. At this time, it can be experimentally and empirically compared with all amino acid sequences contained in the peptide sequence and converted into corresponding values.

The one-hot encoding method may be to convert one or more amino acid sequences contained in the peptide sequence into 0 and 1 at each position.

The BLOSUM processing method can refer to computing the product between the one-hot encoding matrix and the BLOSUM matrix.

The immunogenicity prediction device 100 can input the sequence information of the synthetic long peptide to the model learned from the embedded data to output a first antigen feature value, input the sequence information of the neoantigen peptide to a model learned from the one-hot encoded data to output a second antigen feature value, and input the sequence information of the synthetic long peptide to the model learned from the BLOSUM processed data to output the third antigen feature value.

At this time, the model learned from the embedded data may be a model that is learned by inputting data which is obtained by embedding a plurality of synthetic long peptides and outputting antigenic feature values of the peptides. Antigen feature values of synthetic long peptides used to learn this model may utilize values stored in a database or values acquired through experiments.

The model learned from the one-hot encoded data may be a model that is learned by inputting data which is obtained by one-hot processing a plurality of synthetic long peptides and outputting antigenic feature values of the synthetic long peptides. Antigen feature values of peptides used to learn this model may utilize values stored in a database or values acquired through experiments.

The model learned from BLOSUM-processed data may be a model that is learned by inputting data which is obtained by BLOSUM processing a plurality of synthetic long peptides, and outputting the antigenic feature values of the peptides. Antigen feature values of synthetic long peptides used to learn this model may utilize values stored in a database or values acquired through experiments.

In S130, the immunogenicity prediction device 100 may generate a vector of cleavage probability from the sequence information of synthetic long peptides. The immunogenicity prediction device 100 may output a cleavage feature value using a cleavage prediction model learned by inputting a vector of cleavage probability.

Here, the cleavage prediction model may be a model that is learned from learning data of cleavage feature values through the cleavage probability vectors for each position of synthetic long peptides, and synthetic-length peptides. When synthetic long peptides are injected into the body of a patient, animal, etc., they are cleaved and degraded through predetermined materials present in the body. At this time, the material for cleaving the synthetic long peptides may include proteosome, cathepsin, and the like.

Synthetic long peptides can be cleaved into two or more peptides and bound to HLA class I and/or HLA class II in the body to operate. Some of the cleaved peptides bind to HLA class I, and some of the cleaved peptides can bind to HLA class II.

In S140, the immunogenicity prediction device 100 can input the neoantigen peptide sequence, HLA class I sequence, and HLA class II sequence present in the synthetic long peptide, and output neoantigen feature values related to the immunity and binding affinity to the neoantigen peptide sequence. At this time, the immunogenicity prediction device 100 may utilize a T cell activity data, and a model learned by immunity to peptides.

The immunogenicity prediction device 100 can input synthetic long peptides and HLA class I or II allele sequences as T cell activity data, and output a first neoantigen feature value corresponding to a first neoantigen feature value corresponding to immunity to the neoantigen peptide sequence present in the synthetic long peptide. The immunogenicity prediction device 100 may output first neoantigen feature values corresponding to immunity to neoantigen peptides using a T cell activity data and a model learned by immunity to neoantigen peptides.

The immunogenicity prediction device 100 can input binding data for synthetic long peptide and HLA class I or II, and output second neoantigen feature values corresponding to the binding affinity to neoantigen peptides present in the synthetic long peptides.

The immunogenicity prediction device 100 may output third neoantigen feature values calculated by the product of the first neoantigen feature value and the second neoantigen feature value.

Synthetic long peptides can be cleaved and degraded in a subject and then bound to HLA class I and HLA class II to output a therapeutic effect. The degree to which the peptides present within synthetic long peptides bind to HLA class I or HLA class II can be predicted by a second neoantigen feature value related to the binding affinity. The degree to which the peptides present within the synthetic long peptide increase the immunity of the subject can be predicted by the first neoantigen characteristic value related to the immunity.

In S150, the immunogenicity prediction device 100 may output immunogenicity scores for peptides in consideration of the first to third antigen feature values, the cleavage feature value, and the first to third neoantigen feature values. The immunogenicity score for the peptides may be a value output by inputting first to third antigen feature values, cleavage feature values, and first to third neo-antigen feature values. At this time, the immunogenicity score for the peptides may be determined by a return value for a model learned from the first to third antigen feature values, the cleavage feature value, and the first to third neoantigen feature values. The model learned from the first to third antigen feature values, the cleavage feature value, and the first to third neoantigen feature values is learned by a machine learning method, and can be learned by methods such as supervised learning, unsupervised learning, and reinforced learning.

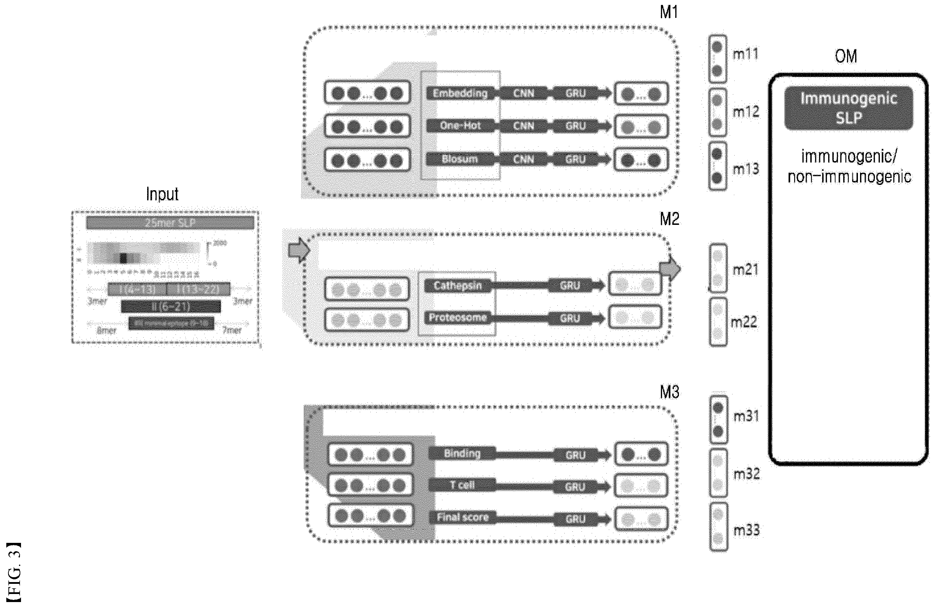

FIG. 3 is an illustrative diagram of a model that outputs immunogenicity scores for synthetic long peptides.

The immunogenicity prediction device 100 according to an embodiment of the present disclosure may include a data input unit for inputting input data, an antigen feature extraction unit M1, a cleavage feature extraction unit M2, a binding affinity prediction unit M3, and a prediction score output unit OM.

The data input unit can input data for synthetic long peptides. The data for synthetic long peptides may include a synthetic long peptide information, a positional information of neoantigen peptides within synthetic long peptides, a cleavage position information within synthetic long peptides, and a data for cleaved peptides. The synthetic long peptide may be a 25 mer peptide (Synthetic Long Peptide, SLP), but is not limited thereto. Here, the synthetic long peptide may contain mutations that leads to the production of neoantigens. The position of the mutation included in the synthetic long peptide can change the position of the neoantigen peptide to the center, in consideration of the main positions at which neoantigen peptides corresponding to HLA class I or class II are found not only in the center but also within synthetic long peptides.

The data used to learn the immunogenicity of synthetic long peptides can be acquired from databases. At this time, among long peptides having the same length as synthetic long peptides used as cancer vaccines acquired from the database, learning can be performed from learning data for immunogenic peptides having information binding to HLA class I or II. In addition, among long peptides of the same length, learning can be performed using learning data for non-immunogenic peptides having information that does not bind to HLA class I or II.

The antigen feature output unit M1 may process synthetic long peptides by processing methods such as embedding, one-hot, and BLOSUM, and output antigen feature values for the processed peptides. The antigen feature output unit M1 may include a first model (CNN, GRU) for outputting a first antigen feature value m11 from the data of the embedded peptides, a second model (CNN, GRU) for outputting a second antigen feature value m12 from data of one-hot processed peptides, and a third model (CNN, GRU) for outputting a third antigen feature value (m13) from data of BLOSUM-processed peptides.

The cleavage feature output unit M2 can input a vector of cleavage probability for each position of a synthetic long peptide and output a cleavage feature value. Synthetic long peptides can be cleaved with enzymes such as proteosome or cathepsin present in the injected subject, wherein the immunogenicity of degraded neoantigen peptides can be altered depending on the cleavage position. Therefore, it is possible to predict immunogenicity considering the cleavage position of synthetic long peptides.

The cleavage feature output unit M2 may output cleavage feature values based on cleavage probability vectors for each position of enzymes present in the subject, e.g., cathepsin and proteosome. More specifically, the cleavage feature output unit M2 can input the cleavage probability vector for each position of the neoantigen peptide from cathepsin, and output the first cleavage feature value m21 through the learned model GRU. The cleavage feature output unit M2 can input the cleavage probability vector for each position of the neoantigen peptides to proteosome, and output the second cleavage feature value m22 through the learned model GRU. The cleavage probability vector for each position for cathepsin and proteosome was illustrated without being limited thereto, and may further include a model that inputs cleavage probability vectors for each position for various materials. In addition, although CNN, GRU and the like are illustrated as learning models, it is not limited thereto, and various learning models can be applied.

The binding affinity output unit M3 may input the neoantigen peptide sequence, HLA class I sequence, and HLA class II sequence within the synthetic long peptides, and output feature values related to the immunity and binding affinity to the neoantigen peptide sequence.

The degree to which the neoantigen peptide within the synthetic long peptide binds to HLA class I and HLA class II, and the degree of immunity of the neoantigen peptide acting after binding can be output using a model that predicts one or more bonding forces, a model that predicts one or more immunity, and the like.

More specifically, the immune avidity output unit M3 can input binding data on the binding relationship between the neoantigen peptide and HLA alleles within the synthetic long peptides, and output one feature value m31 related to the binding affinity using the learned model CNN-GRU.

The binding affinity output unit M3 can input a T cell activity data for neoantigen peptide sequences within synthetic long peptides and/or HLA class I and II allele sequences, and can output the second feature value m32 corresponding to the immunity of the neoantigen peptide using the learned model.

The binding affinity output unit M3 may output a third neoantigen feature value m33 obtained by multiplying the first neoantigen feature value m31 and the second neoantigen feature value m32.

The immunogenicity score output unit OM can input the values output from M1 (m11, m12, m13), the values output from M2 (m21, m22), and the values output from M3 (m31, m32, m33) to output immunogenicity scores for synthetic long peptides.

If values, such as synthetic long peptides, mutation position within synthetic long peptides, values corresponding to the cleavage probability for each position of synthetic long peptides, the degree of binding of neoantigen peptides within synthetic long peptides, and immunity level are entered, it can be designed to output an immunogenicity score for synthetic long peptides accordingly. Immunogenicity scores can be output as immunogenic and non-immunogenic values. If the values output from M1 (m11, m12, m13), the values output from M2 (m21, m22), and the values output from M3 (m31, m32, m33) are input to the immunogenicity score output unit (OM), immunogenic and non-immunogenic values can be output as a combination of values output through hidden layers.

The models may be models learned by methods such as supervised learning, unsupervised learning, self-supervised learning, clustering, and anomaly detection.

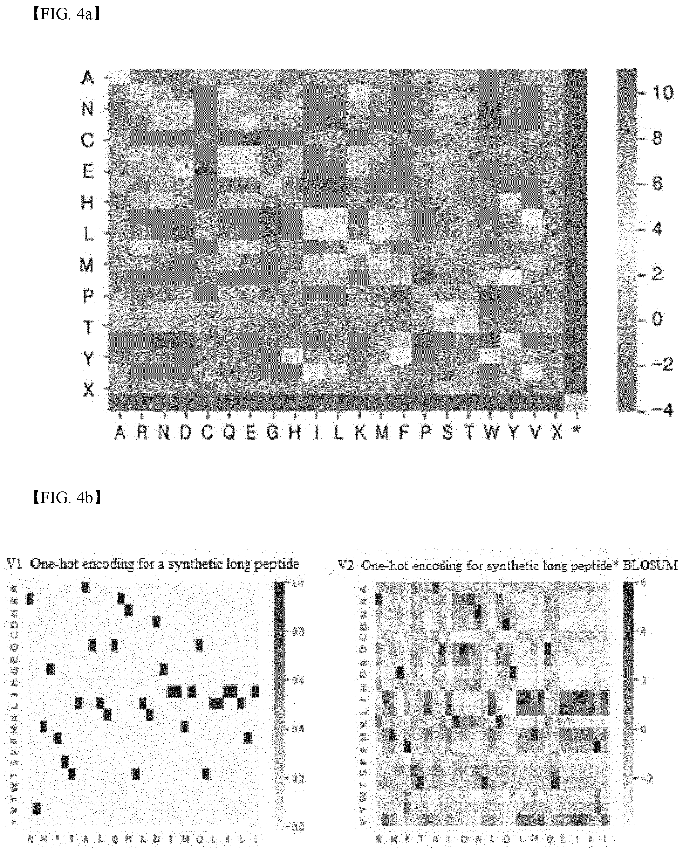

FIG. 4a is an illustrative diagram of a BLOSUM matrix used in the immunogenicity prediction device 100.

FIG. 4b is an illustrative diagram which is encoded by one-hot encoding V1 and product V2 between one-hot encoding and BLOSUM matrix for a synthetic long peptide sequence based on the BLOSUM matrix of FIG. 4a.

By the method described above, the synthetic long peptide sequence encoding is input to one or more CNNs to extract feature values, and these feature values can be learned by GRU and derived by the encoder.

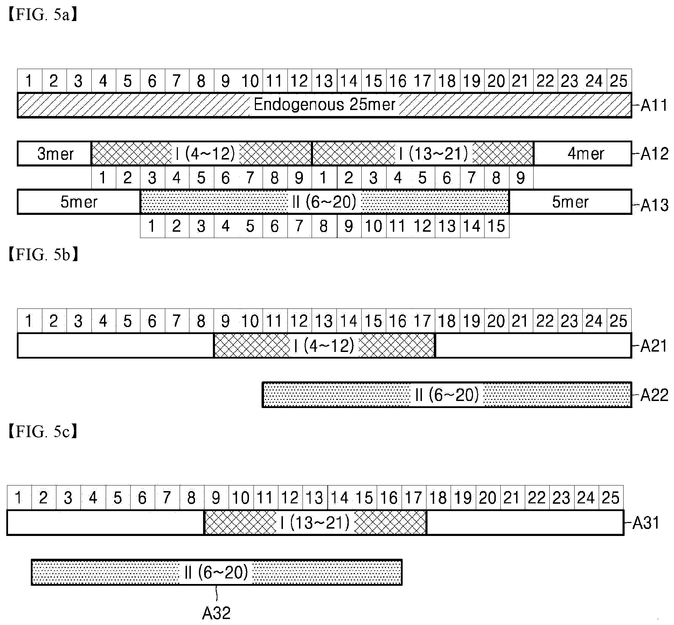

FIG. 5a is an illustrative diagram of the main positions of the synthetic long peptides corresponding to HLA classes I and II, among neoantigen peptides present in the synthetic long peptides.

The synthetic long peptide A11 may have a length of 25mer as shown in the figure. The synthetic long peptide A11 can be cleaved as shown in A12 and A13 and bound to HLA class I or II, etc. Neoantigen peptides corresponding to HLA class I are mainly found in the first portion (4 to 12) and the second portion (13 to 21) in the synthetic long peptide. Neoantigen peptides corresponding to HLA class II are mainly found in the third portion (6 to 20) of the synthetic long peptides. The synthetic long peptide A11 injected into the subject may be cleaved as shown in A12 and A13 by a cleavage enzyme present in the subject, and then bound to HLA class I or II to operate. If the neoantigen peptide corresponding to HLA class I is arranged at the center of the 25mer, it can be seen that the epitope corresponding to HLA class II is well conserved.

FIG. 5b is an illustrative diagram of neoantigen peptides corresponding to HLA classes I and II within synthetic long peptides arranged in an alternative manner.

As shown by A21, in the case of a 25mer where a neoantigen peptide A21 corresponding to HLA class I arranged on the left side within the synthetic long peptide is arranged at the center, the class II epitope can be arranged within the length of synthetic long peptides, thus enhancing the immune effect due to cross-presentation.

FIG. 5c is an illustrative diagram of neoantigen peptides arranged in an alternative manner.

As shown by A31, in the case of a 25mer where a neoantigen peptide A21 corresponding to HLA class I arranged on the left side within the synthetic long peptide is arranged at the center, the class II epitope can be arranged within the length of synthetic long peptides, thus enhancing the immune effect due to cross-presentation.

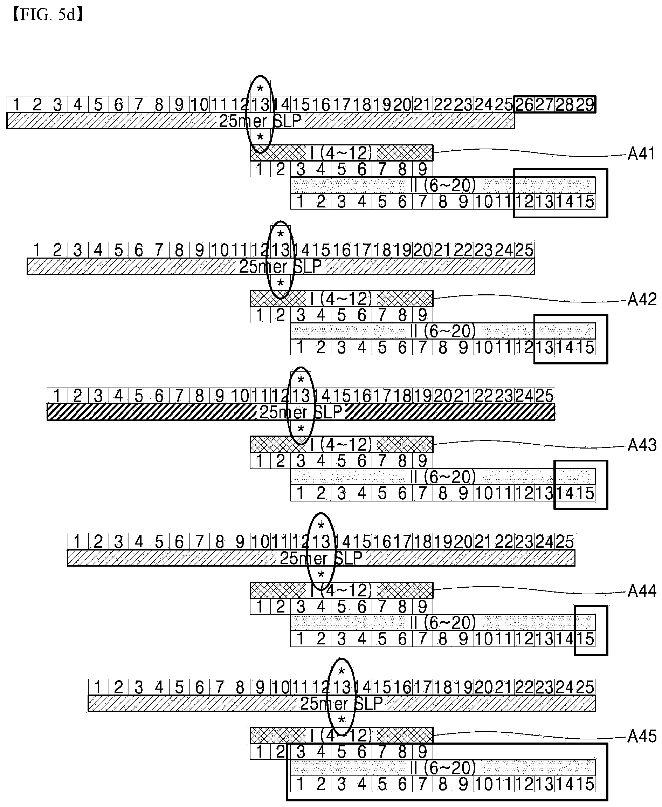

FIG. 5d is an illustrative diagram of neoantigen peptides arranged in an alternative manner.

Mutation arrangement information within the synthetic long peptide may be changeable to the first to fifth or more positions of the neoantigen peptide corresponding to HLA class I.

Mutation of the neoantigen peptide corresponding to HLA class I within the synthetic long peptides can be changed, as shown by A41, to the first position within the neoantigen peptides and the center of the synthetic long peptide. In this case, the epitope binding to HLA class II does not exist within the synthetic long peptide length but is cleaved so that cross-presentation does not occur.

Mutation of the neoantigen peptide corresponding to HLA class I within the synthetic long peptides can be changed, as shown by A42, to the second position within the neoantigen peptides and the center of the synthetic long peptides. In this case, the epitope binding to HLA class II does not exist within the synthetic long peptide length but is cleaved so that cross-presentation does not occur.

Mutation of the neoantigen peptide corresponding to HLA class I within the synthetic long peptides can be changed, as shown by A43, to the third position within the neoantigen peptides and the center of the synthetic long peptides. In this case, the epitope binding to HLA class II does not exist within the synthetic long peptide length but is cleaved so that cross-presentation does not occur.

Mutation of the neoantigen peptide corresponding to HLA class I within the synthetic long peptides can be changed, as shown by A44, to the fourth position within the neoantigen peptide and the center of the synthetic long peptides. In this case, the epitope binding to HLA class II does not exist within the synthetic long peptide length but is cleaved so that cross-presentation does not occur.

Mutation of the neoantigen peptide corresponding to HLA class I within the synthetic long peptides can be changed, as shown by A45, to the fifth position within the neoantigen peptide and the center of the synthetic long peptides. In this case, the epitope binding to HLA class II exists within the synthetic long peptide length, after which cross-presentation is possible. That is, the mutation position within the synthetic long peptides may be designed so as to be arranged at one of the positions where the epitope corresponding to HLA class II is not cleaved while being arranged at a position other than the center.

The immunogenicity prediction device 100 according to an embodiment of the present disclosure can predict immunogenicity due to neoantigen peptides and mutations corresponding to HLA classes I and II within synthetic long peptides.

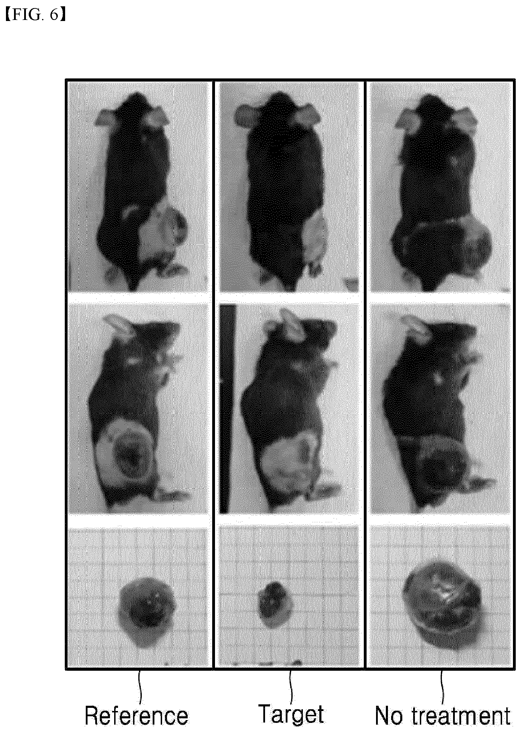

FIG. 6 is a diagram showing the results of inhibiting cancer growth by treating a melanoma mouse model with a synthetic long peptide found by an immunogenicity prediction device.

It can be seen that the results of treating the same carcinoma subject with the synthetic long peptide having the highest immunogenicity score with the immunogenicity prediction device 100 inhibit cancer growth as in Target. It can be seen that the results of treating the same carcinoma subject with a different method, i.e., a synthetic long peptide in which a mutation is arranged at the center inhibit cancer growth as in Reference, but is not as effective as compared with the result of the present disclosure. The results of not giving any treatment to the same carcinoma subject increase in the size of cancer cells, which accelerates cancer growth, as in No treatment. As shown in FIG. 6, it can be seen that the cancer growth inhibitory effect when treated with the synthetic long peptide determined by the immunogenicity prediction device 100 is much higher than that of other methods, that is, the synthetic long peptide in which mutation is arranged at the center.

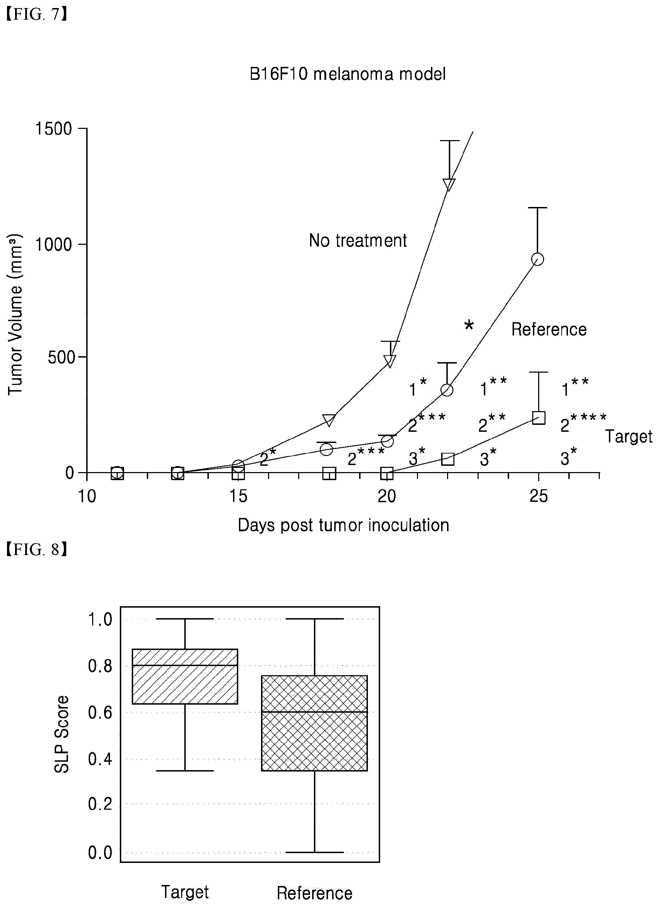

FIG. 7 is an illustrative diagram of a graph of cancer size change over time in a melanoma B16F10 mouse model.

As shown in FIG. 7, if not treated at all, the size of the cancer in the melanoma B16F10 mouse model has 500 mm³ on day 20, whereas if treated with the synthetic long peptide determined according to the embodiments of the present disclosure, it is measured to have 100 mm³ on day 20. That is, if treated with the synthetic long peptide determined according to the embodiments of the present disclosure, it can be seen that the size of the tumor is much smaller than that of other comparative subjects, which is greatly inhibited in cancer growth.

FIG. 8 is a diagram that presents the immunogenicity prediction score for the existing method for five synthetic long peptides (SLPs) injected into B16F10 melanoma mice, i.e., a method considering cross-presentation relative to a method in which mutation is arranged at the center (Reference), i.e., a method in which the neoantigen corresponding to HLA class I is arranged at the center (Target). As shown in FIG. 8, for the 5 types of synthetic long peptides injected into melanoma mice, the input values for the existing method, that is, the synthetic long peptide (Reference) in which the mutation is arranged at the center, are input into the data input unit 110 of the immunogenicity prediction device, and then the immunogenicity score can be predicted through the immunogenicity score output unit 130. In addition, for the same melanoma mouse, the input value for a synthetic long peptide (Target) in which the neoantigen peptide corresponding to HLA class I is arranged at the center is input into the data input unit 110 of the immunogenicity prediction device, and then immunogenicity scores can be predicted through the score output unit 130. Comparing predicted immunogenicity scores derived by different methods with each other, it can be seen that the immunogenicity score for the synthetic long peptide placed in the center of the neoantigen, which had a large cancer growth inhibitory effect, is predicted to be much higher.

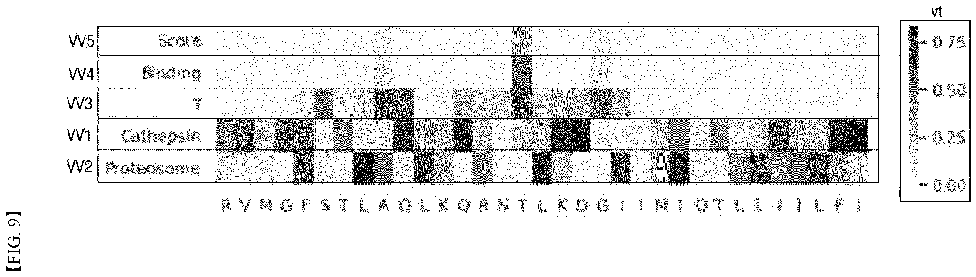

FIG. 9 is an illustrative diagram of vectors input to the immunogenicity prediction device 100.

A vector input to the immunogenicity prediction device may be as shown in FIG. 9. At this time, an image displayed in a color corresponding to each value may be generated and input to the immunogenicity prediction device. As shown in vvt, darker colors represent higher probability values. The probability vector VV1 for each position cleaved by cathepsin and the probability vector VV2 for each position cleaved by proteosome may be data input to the cleavage feature output unit 122. Additionally, a vector VV4 showing the magnitude of binding affinity with HLA for the neoantigen peptide present in the synthetic long peptide, a vector VV5 showing the magnitude of immunogenicity for T cell activity, and a vector VV3 that is the product of VV4 and VV5 may be data for training the binding affinity output unit 123, but is not limited thereto and the binding affinity output unit 123 may be trained with various vectors.

A vector input to the immunogenicity prediction device may be as shown in FIG. 4b. At this time, an image shown in a color corresponding to each value may be generated and input to the immunogenicity prediction device. As shown in vvt, darker colors represent higher probability values. A one-hot encoding matrix VV1 for the synthetic long peptide sequence and a matrix VV2 obtained by multiplying the one-hot encoding matrix and the BLOSUM matrix may be data for training the antigen feature output unit 121.

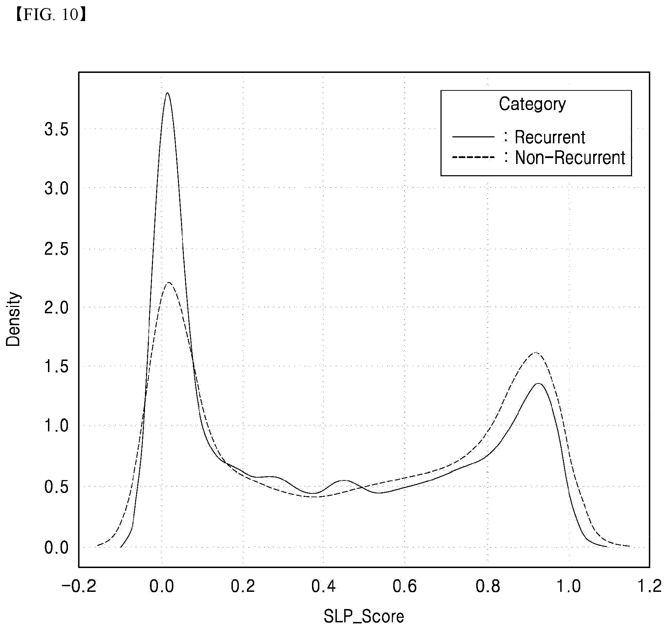

FIG. 10 shows that synthetic long peptides derived from frequently observed mutations (Recurrent variants) and rarely observed mutations (Non-recurrent variants) between patients in the cancer database TCGA (The Cancer Genome Atlas) are predicted with the immunogenicity prediction device of the present disclosure, and as a result, the immunogenicity score of the frequently observed mutations is significantly lower than that of mutations observed only once (p-value=1.17e-182). That is, according to the present disclosure, since mutations with low immunogenicity and synthetic long peptides derived therefrom are easily evaded from the patient's immune system, the mutations do not disappear but remain, which exhibits the frequently observed immunoediting phenomenon. The x-axis represents the immunogenicity score, and the y-axis represents the density of the number of times each score occurs.

The device described herein may be implemented using hardware components, software components, and/or a combination of the hardware components and the software components. For example, the device and the components described in the embodiments may be implemented using one or more general-purpose or special purpose computers, such as, for example, a processor, a controller and an ALU (arithmetic logic unit), a digital signal processor, a microcomputer, a FPGA (field programmable array), a PLU (programmable logic unit), a microprocessor or any other device capable of responding to and executing instructions in a defined manner. The processing device may run an operating system (OS) and one or more software applications that run on the operating system. The processing device also may access, store, manipulate, process, and create data in response to execution of the software. For purpose of simplicity, the description of a processing device is used as singular; however, one skilled in the art will be appreciated that a processing device may include multiple processing elements and/or multiple types of processing elements. For example, a processing device may include multiple processors or a processor and a controller. In addition, different processing configurations are possible, such as a parallel processor.

The software may include a computer program, a piece of code, an instruction, or some combination thereof, to independently or collectively instruct or configure the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums.

The methods according to the above-described embodiments may be recorded in non-transitory computer-readable media including program instructions to implement various operations embodied by a computer. The computer-readable media may also include, alone or in combination with the program instructions, data files, data structures, and the like. The program instructions recorded on the media may be those specially designed and constructed for the purposes of the embodiments, or they may be of the kind well-known and available to those having skill in the computer software arts. Examples of the computer-readable media include magnetic media such as hard disks, floppy disks, and magnetic tape; optical media such as CD ROM disks and DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described embodiments, or vice versa.

Although embodiments of the present disclosure have been described in detail above with reference to the limited embodiments and the accompanying drawings various modifications and variations can be made by those skilled in the art. For example, suitable results may be achieved if the described techniques are performed in a different order, and/or if components in a described system, architecture, device, or circuit are coupled or combined in a different manner and/or replaced or supplemented by other components or their equivalents.

Therefore, the scope of the disclosure is defined not by the detailed description, but by the claims and all variations within the scope of the claims are to be construed as being included in the disclosure

## Claims

1. An immunogenicity prediction method comprising the steps of:
acquiring information about synthetic long peptides for treating carcinoma in a subject through an immunogenicity prediction device;
processing data obtained from the information about synthetic long peptides, using at least one of embedding, one-hot encoding, and BLOSUM through the immunogenicity prediction device to output one or more antigen feature values;
generating a vector of cleavage probability based on sequence information of the synthetic long peptides through the immunogenicity prediction device;
inputting the cleavage probability vector for each position of the synthetic long peptides through the immunogenicity prediction device to output one or more cleavage feature values, using a model trained with a cleavage probability vector as an input and one or more cleavage feature values as an output;
inputting neoantigen peptide sequences corresponding to an HLA class I sequence and an HLA class II sequence within the synthetic long peptides through the immunogenicity prediction device to output one or more neoantigen feature values related to the immunity and binding affinity present in the synthetic long peptide, using a model trained with a neoantigen peptide sequence, an HLA class I sequence and an HLA class II sequence as inputs and one or more neoantigen feature values as an output; and
outputting an immunogenicity score of the synthetic long peptide through the immunogenicity prediction device, using a model trained with the one or more antigen feature values, the one or more cleavage feature values, and the one or more neoantigen feature values as inputs and an immunogenicity score as an output.

2. The immunogenicity prediction method according to claim 1, wherein:
the cleavage probability vector for each position of the neoantigen peptide is a cleavage probability vector for each position, when a synthetic long peptide is cleaved with proteosomes or cathepsins, which are cleavage enzymes present in the subject.

3. The immunogenicity prediction method according to claim 1, wherein:
the step of outputting neoantigen feature values related to the immunity and binding affinity comprises,
outputting a first neoantigen feature value related to the immunity using a T cell activity data and a model learned with immunity to neoantigen peptides,
outputting a second neoantigen feature value corresponding to the binding of the neoantigen peptide present in the synthetic long peptide using a model learned by inputting the binding data for the neoantigen peptide and HLA classes I and II, and
outputting a third neoantigen feature value that is a product of the first neoantigen feature value and the second neoantigen feature value.

4. The immunogenicity prediction method according to claim 1, wherein:
the step of outputting the one or more antigen feature values comprises,
outputting a first antigen feature value using a model learned from data obtained by embedding the sequence information of the synthetic long peptide,
outputting a second antigen feature value using a model learned from data obtained by one-hot encoding the sequence information of the synthetic long peptide,
and
outputting a third antigen feature value using a model learned from data obtained by BLOSUM processing the sequence information of the synthetic long peptide.

5. The immunogenicity prediction method according to claim 1, wherein:
the synthetic long peptide has a length of 40mers or less.

6. The immunogenicity prediction method according to claim 1, wherein:
the synthetic long peptide is formed such that a neoantigen peptide sequence corresponding to HLA class I is positioned at the center.

7. The immunogenicity prediction method according to claim 1, further comprising:
determining the synthetic long peptide with the highest immunogenicity score for a plurality of synthetic long peptides by repeatedly performing the step of outputting the one or more antigen feature values, the step of outputting the one or more cleavage feature values, the step of outputting the one or more neoantigen feature values, and the step of outputting the immunogenicity score.

8. A computer program stored on a computer-readable storage medium to execute the method of any one of claims 1 to 7 using a computer.

9. An immunogenicity prediction device, comprising:
a data input unit that acquires information about synthetic long peptides for treating carcinoma in a subject;
an antigen feature output unit that processes data obtained from the information about synthetic long peptides, using at least one of embedding, one-hot encoding, and BLOSUM to output one or more antigen feature values;
a cleavage feature extraction unit that generates a vector of cleavage probability based on sequence information of the synthetic long peptides
a cleavage feature output unit that inputs the cleavage probability vector for each position of the synthetic long peptide to output one or more cleavage feature values, using a model trained with a cleavage probability vector as an input and one or more cleavage feature values as an output;
a binding affinity output unit that inputs neoantigen peptide sequences corresponding to an HLA class I sequence and an HLA class II sequence within the synthetic long peptides to output one or more neoantigen feature values related to the immunity and binding affinity present in the synthetic long peptide, using a model trained with a neoantigen peptide sequence, an HLA class I sequence and an HLA class II sequence as inputs and one or more neoantigen feature values as an output; and
an immunogenicity score output unit that outputs an immunogenicity score of the synthetic long peptide, using a model trained with the one or more antigen feature values, the one or more cleavage feature values, and the one or more neoantigen feature values as inputs and an immunogenicity score as an output.

10. The immunogenicity prediction device according to claim 9, wherein:
the cleavage probability vector for each position of the synthetic long peptide is a cleavage probability vector for each position, when a synthetic long peptide is cleaved with proteosomes or cathepsins, which are cleavage enzymes present in the subject.

11. The immunogenicity prediction device according to claim 9, wherein:
the binding affinity output unit
outputs a first neoantigen feature value related to the immunity using a T cell activity data and a model learned with immunity to neoantigen peptides,
outputs a second neoantigen feature value corresponding to the binding of the neoantigen peptide present in the synthetic long peptide using the model learned by inputting the binding data for the neoantigen peptide and HLA classes I and II, and
outputs a third neoantigen feature value that is a product of the first neoantigen feature value and the second neoantigen feature value.

12. The immunogenicity prediction device according to claim 9, wherein:
the antigen feature output unit
outputs a first antigen feature value using a model learned by embedding the sequence information of the synthetic long peptide,
outputs a second antigen feature value using a model learned from data obtained by one-hot encoding the sequence information of the synthetic long peptide, and
outputs a third antigen feature value using a model learned from data obtained by BLOSUM processing the sequence information of the synthetic long peptide.

13. The immunogenicity prediction device according to claim 9, wherein:
the neoantigen peptide has a length of 40mers or less.

14. The immunogenicity prediction device according to claim 9, wherein:
the synthetic long peptide is formed such that a neoantigen peptide sequence corresponding to HLA class I is positioned at the center.

## Patentansprüche

1. Verfahren zur Vorhersage der Immunogenität, umfassend die folgenden Schritte:
Erfassen von Informationen über synthetische lange Peptide zur Behandlung von Karzinomen bei einem Patienten mittels einer Vorrichtung zur Vorhersage der Immunogenität;
Verarbeiten von Daten, die aus den Informationen über synthetische lange Peptide gewonnen wurden, unter Verwendung von mindestens einem der Verfahren Einbettung, One-Hot-Kodierung und BLOSUM mittels der Vorrichtung zur Vorhersage der Immunogenität, um einen oder mehrere Antigen-Merkmalswerte auszugeben;
Erzeugen eines Vektors der Spaltwahrscheinlichkeit auf der Grundlage von Sequenzinformationen der synthetischen langen Peptide mittels der Vorrichtung zur Vorhersage der Immunogenität;
Eingeben des Spaltwahrscheinlichkeitsvektors für jede Position der synthetischen langen Peptide über die Vorrichtung zur Vorhersage der Immunogenität, um einen oder mehrere Spaltmerkmalswerte auszugeben, unter Verwendung eines Modells, das mit einem Spaltwahrscheinlichkeitsvektor als Eingabe und einem oder mehreren Spaltmerkmalswerten als Ausgabe trainiert wurde;
Eingeben von Neoantigen-Peptidsequenzen, die einer HLA-Klasse-I-Sequenz und einer HLA-Klasse-II-Sequenz innerhalb der synthetischen langen Peptide entsprechen, über die Immunogenitätsvorhersagevorrichtung, um ein oder mehrere Neoantigen-Merkmalswerte auszugeben, die sich auf die im synthetischen langen Peptid vorhandene Immunität und Bindungsaffinität beziehen, unter Verwendung eines Modells, das mit einer Neoantigen-Peptidsequenz, einer HLA-Klasse-I-Sequenz und einer HLA-Klasse-II-Sequenz als Eingaben und einem oder mehreren Neoantigen-Merkmalswerten als Ausgabe trainiert wurde; und
Ausgeben eines Immunogenitätswerts des synthetischen langen Peptids durch die Immunogenitätsvorhersagevorrichtung unter Verwendung eines Modells, das mit dem einen oder den mehreren Antigen-Merkmalswerten, dem einen oder den mehreren Spaltungs-Merkmalswerten und dem einen oder den mehreren Neoantigen-Merkmalswerten als Eingaben und einem Immunogenitätswert als Ausgabe trainiert wurde.

2. Verfahren zur Vorhersage der Immunogenität nach Anspruch 1, wobei:
der Spaltwahrscheinlichkeitsvektor für jede Position des Neoantigen-Peptids ein Spaltwahrscheinlichkeitsvektor für jede Position ist, wenn ein synthetisches langes Peptid mit Proteosomen oder Kathepsinen gespalten wird, bei denen es sich um im Probanden vorhandene Spaltenzyme handelt.

3. Verfahren zur Vorhersage der Immunogenität gemäß Anspruch 1, wobei:
der Schritt des Ausgebens von Neoantigen-Merkmalswerten, die sich auf die Immunität und die Bindungsaffinität beziehen, umfasst
das Ausgeben eines ersten Neoantigen-Merkmalswerts, der sich auf die Immunität bezieht, unter Verwendung von T-Zell-Aktivitätsdaten und eines Modells, das mit der Immunität gegenüber Neoantigen-Peptiden trainiert wurde,
Ausgeben eines zweiten Neoantigen-Merkmalswerts, der der Bindung des in dem synthetischen langen Peptid vorhandenen Neoantigen-Peptids entspricht, unter Verwendung eines Modells, das durch Eingabe der Bindungsdaten für das Neoantigen-Peptid und die HLA-Klassen I und II trainiert wurde, und
Ausgeben eines dritten Neoantigen-Merkmalswerts, der ein Produkt des ersten Neoantigen-Merkmalswerts und des zweiten Neoantigen-Merkmalswerts ist.

4. Verfahren zur Vorhersage der Immunogenität nach Anspruch 1, wobei:
der Schritt des Ausgebens des einen oder der mehreren Antigen-Merkmalswerte umfasst,
das Ausgeben eines ersten Antigen-Merkmalswerts unter Verwendung eines Modells, das aus Daten trainiert wurde, die durch Einbetten der Sequenzinformation des synthetischen langen Peptids erhalten wurden,
Ausgeben eines zweiten Antigen-Merkmalswerts unter Verwendung eines Modells, das aus Daten gelernt wurde, die durch One-Hot-Kodierung der Sequenzinformation des synthetischen langen Peptids erhalten wurden,
und
Ausgeben eines dritten Antigenmerkmalswerts unter Verwendung eines Modells, das aus Daten gelernt wurde, die durch BLOSUM-Verarbeitung der Sequenzinformationen des synthetischen langen Peptids erhalten wurden.

5. Verfahren zur Vorhersage der Immunogenität nach Anspruch 1, wobei:
das synthetische lange Peptid eine Länge von 40 Mer oder weniger aufweist.

6. Verfahren zur Vorhersage der Immunogenität gemäß Anspruch 1, wobei:
das synthetische lange Peptid so gebildet ist, dass eine HLA-Klasse-I-entsprechende Neoantigen-Peptidsequenz in der Mitte positioniert ist.

7. Verfahren zur Vorhersage der Immunogenität nach Anspruch 1, das ferner umfasst:
Bestimmen des synthetischen langen Peptids mit dem höchsten Immunogenitätswert für eine Vielzahl von synthetischen langen Peptiden durch wiederholtes Ausführen des Schritts des Ausgebens des einen oder der mehreren Antigen-Merkmalswerte, des Schritts des Ausgebens des einen oder der mehreren Spaltungs-Merkmalswerte, des Schritts des Ausgebens des einen oder der mehreren Neoantigen-Merkmalswerte und des Schritts des Ausgebens des Immunogenitätswerts.

8. Ein auf einem computerlesbaren Speichermedium gespeichertes Computerprogramm zur Ausführung des Verfahrens nach einem der Ansprüche 1 bis 7 unter Verwendung eines Computers.

9. Vorrichtung zur Vorhersage der Immunogenität, umfassend:
eine Dateneingabeeinheit, die Informationen über synthetische lange Peptide zur Behandlung von Karzinomen bei einem Probanden erfasst;
eine Antigenmerkmal-Ausgabeeinheit, die Daten verarbeitet, die aus den Informationen über synthetische lange Peptide gewonnen wurden, wobei mindestens eines von Einbettung, One-Hot-Kodierung und BLOSUM verwendet wird, um einen oder mehrere Antigenmerkmalwerte auszugeben;
eine Spaltmerkmalextraktionseinheit, die auf der Grundlage von Sequenzinformationen der synthetischen langen Peptide einen Vektor der Spaltwahrscheinlichkeit erzeugt;
eine Spaltmerkmals-Ausgabeeinheit, die den Spaltwahrscheinlichkeitsvektor für jede Position des synthetischen langen Peptids eingibt, um einen oder mehrere Spaltmerkmalswerte auszugeben, wobei ein Modell verwendet wird, das mit einem Spaltwahrscheinlichkeitsvektor als Eingabe und einem oder mehreren Spaltmerkmalswerten als Ausgabe trainiert wurde;
eine Bindungsaffinitäts-Ausgabeeinheit, die Neoantigen-Peptidsequenzen eingibt, die einer HLA-Klasse-I-Sequenz und einer HLA-Klasse-II-Sequenz innerhalb der synthetischen langen Peptide entsprechen, um einen oder mehrere Neoantigen-Merkmalswerte auszugeben, die sich auf die im synthetischen langen Peptid vorhandene Immunität und Bindungsaffinität beziehen, unter Verwendung eines Modells, das mit einer Neoantigen-Peptidsequenz, einer HLA-Klasse-I-Sequenz und einer HLA-Klasse-II-Sequenz als Eingaben und einem oder mehreren Neoantigen-Merkmalswerten als Ausgabe trainiert wurde; und
eine Immunogenitäts-Score-Ausgabeeinheit, die einen Immunogenitäts-Score des synthetischen langen Peptids ausgibt, unter Verwendung eines Modells, das mit dem einen oder den mehreren Antigen-Merkmalswerten, dem einen oder den mehreren Spaltungs-Merkmalswerten und dem einen oder den mehreren Neoantigen-Merkmalswerten als Eingaben und einem Immunogenitäts-Score als Ausgabe trainiert wurde.

10. Vorrichtung zur Vorhersage der Immunogenität nach Anspruch 9, wobei:
der Spaltwahrscheinlichkeitsvektor für jede Position des synthetischen langen Peptids ein Spaltwahrscheinlichkeitsvektor für jede Position ist, wenn ein synthetisches langes Peptid mit Proteosomen oder Kathepsinen gespalten wird, bei denen es sich um im Probanden vorhandene Spaltenzyme handelt.

11. Vorrichtung zur Vorhersage der Immunogenität gemäß Anspruch 9, wobei:
die Bindungsaffinitäts-Ausgabeeinheit
einen ersten Neoantigen-Merkmalswert ausgibt, der sich auf die Immunität bezieht, unter Verwendung von T-Zell-Aktivitätsdaten und eines Modells, das mit der Immunität gegenüber Neoantigen-Peptiden trainiert wurde,
einen zweiten Neoantigen-Merkmalswert ausgibt, der der Bindung des im synthetischen langen Peptid vorhandenen Neoantigen-Peptids entspricht, unter Verwendung des Modells, das durch Eingabe der Bindungsdaten für das Neoantigen-Peptid und die HLA-Klassen I und II trainiert wurde, und
einen dritten Neoantigen-Merkmalswert ausgibt, der ein Produkt aus dem ersten Neoantigen-Merkmalswert und dem zweiten Neoantigen-Merkmalswert ist.

12. Vorrichtung zur Vorhersage der Immunogenität nach Anspruch 9, wobei:
die Antigenmerkmal-Ausgabeeinheit
einen ersten Antigenmerkmalswert unter Verwendung eines Modells ausgibt, das durch Einbettung der Sequenzinformation des synthetischen langen Peptids trainiert wurde,
einen zweiten Antigenmerkmalswert unter Verwendung eines Modells ausgibt, das aus Daten gelernt wurde, die durch One-Hot-Kodierung der Sequenzinformation des synthetischen langen Peptids erhalten wurden, und
einen dritten Antigenmerkmalswert unter Verwendung eines Modells ausgibt, das aus Daten gelernt wurde, die durch BLOSUM-Verarbeitung der Sequenzinformationen des synthetischen langen Peptids erhalten wurden.

13. Vorrichtung zur Vorhersage der Immunogenität nach Anspruch 9, wobei:
das Neoantigen-Peptid eine Länge von 40 Mer oder weniger aufweist.

14. Vorrichtung zur Vorhersage der Immunogenität gemäß Anspruch 9, wobei:
das synthetische lange Peptid so gebildet ist, dass eine HLA-Klasse-I-entsprechende Neoantigen-Peptidsequenz in der Mitte positioniert ist.

## Revendications

1. Procédé de prédiction de l'immunogénicité comprenant les étapes suivantes :
l'acquisition d'informations sur des peptides longs synthétiques destinés au traitement d'un carcinome chez un sujet à l'aide d'un dispositif de prédiction de l'immunogénicité ;
traiter les données obtenues à partir des informations sur les peptides synthétiques longs, en utilisant au moins l'une des techniques suivantes : l'encodage par imbrication, l'encodage one-hot et BLOSUM, via le dispositif de prédiction de l'immunogénicité, afin de générer une ou plusieurs valeurs de caractéristiques antigéniques ;
générer un vecteur de probabilité de clivage sur la base des informations de séquence des peptides synthétiques longs à l'aide du dispositif de prédiction d'immunogénicité ;
entrer le vecteur de probabilité de clivage pour chaque position des peptides synthétiques longs via le dispositif de prédiction d'immunogénicité afin de générer une ou plusieurs valeurs de caractéristiques de clivage, en utilisant un modèle entraîné avec un vecteur de probabilité de clivage en entrée et une ou plusieurs valeurs de caractéristiques de clivage en sortie ;
saisir des séquences de peptides néoantigènes correspondant à une séquence HLA de classe I et à une séquence HLA de classe II au sein des peptides longs synthétiques via le dispositif de prédiction de l'immunogénicité afin de générer une ou plusieurs valeurs caractéristiques de néoantigène liées à l'immunité et à l'affinité de liaison présentes dans le peptide long synthétique, en utilisant un modèle entraîné avec une séquence de peptide néoantigène, une séquence HLA de classe I et une séquence HLA de classe II en entrée et une ou plusieurs valeurs caractéristiques de néoantigène en sortie ; et
la production d'un score d'immunogénicité du long peptide synthétique par le dispositif de prédiction d'immunogénicité, en utilisant un modèle entraîné avec la ou les valeurs de caractéristiques d'antigène, la ou les valeurs de caractéristiques de clivage et la ou les valeurs de caractéristiques de néoantigène en tant qu'entrées, et un score d'immunogénicité en tant que sortie.

2. Procédé de prédiction de l'immunogénicité selon la revendication 1, dans lequel :
le vecteur de probabilité de clivage pour chaque position du peptide néoantigénique est un vecteur de probabilité de clivage pour chaque position, lorsqu'un long peptide synthétique est clivé par des protéosomes ou des cathepsines, qui sont des enzymes de clivage présentes chez le sujet.

3. Procédé de prédiction de l'immunogénicité selon la revendication 1, dans lequel :
l'étape consistant à fournir des valeurs de caractéristiques de néoantigènes liées à l'immunité et à l'affinité de liaison comprend :
la génération d'une première valeur caractéristique du néoantigène liée à l'immunité à l'aide de données d'activité des lymphocytes T et d'un modèle appris avec l'immunité aux peptides néoantigènes,
la génération d'une deuxième valeur caractéristique du néoantigène correspondant à la liaison du peptide néoantigène présent dans le long peptide synthétique à l'aide d'un modèle appris en entrant les données de liaison pour le peptide néoantigène et les classes HLA I et II, et
la génération d'une troisième valeur de caractéristique de néoantigène qui est le produit de la première valeur de caractéristique de néoantigène et de la deuxième valeur de caractéristique de néoantigène.

4. Procédé de prédiction de l'immunogénicité selon la revendication 1, dans lequel :
l'étape consistant à générer la ou les valeurs de caractéristique d'antigène comprend :
la production d'une première valeur de caractéristique d'antigène à l'aide d'un modèle appris à partir de données obtenues en intégrant les informations de séquence du long peptide synthétique,
la génération d'une deuxième valeur de caractéristique d'antigène à l'aide d'un modèle appris à partir de données obtenues par codage one-hot des informations de séquence du long peptide synthétique,
et
la génération d'une troisième valeur de caractéristique antigénique à l'aide d'un modèle appris à partir de données obtenues par traitement BLOSUM des informations de séquence du long peptide synthétique.

5. Procédé de prédiction de l'immunogénicité selon la revendication 1, dans lequel :
le long peptide synthétique a une longueur de 40 mères ou moins.

6. Procédé de prédiction de l'immunogénicité selon la revendication 1, dans lequel :
le long peptide synthétique est formé de telle sorte qu'une séquence peptidique néoantigénique correspondant à HLA de classe I soit positionnée au centre.

7. Procédé de prédiction de l'immunogénicité selon la revendication 1, comprenant en outre :
la détermination du long peptide synthétique présentant le score d'immunogénicité le plus élevé parmi une pluralité de longs peptides synthétiques, en effectuant de manière répétée l'étape consistant à fournir une ou plusieurs valeurs caractéristiques d'antigène, l'étape consistant à fournir une ou plusieurs valeurs caractéristiques de clivage, l'étape consistant à fournir une ou plusieurs valeurs caractéristiques de néoantigène, et l'étape consistant à fournir le score d'immunogénicité.

8. Programme informatique stocké sur un support de stockage lisible par ordinateur pour exécuter le procédé selon l'une quelconque des revendications 1 à 7 à l'aide d'un ordinateur.

9. Dispositif de prédiction de l'immunogénicité, comprenant :
une unité d'entrée de données qui acquiert des informations sur des peptides longs synthétiques destinés au traitement d'un carcinome chez un sujet ;
une unité de sortie de caractéristiques antigéniques qui traite les données obtenues à partir des informations concernant les peptides synthétiques longs, en utilisant au moins l'une des techniques suivantes : l'encodage par imbrication, l'encodage one-hot et BLOSUM, afin de fournir en sortie une ou plusieurs valeurs de caractéristiques antigéniques ;
une unité d'extraction de caractéristiques de clivage qui génère un vecteur de probabilité de clivage sur la base d'informations de séquence des peptides longs synthétiques ;
une unité de sortie de caractéristiques de clivage qui reçoit en entrée le vecteur de probabilité de clivage pour chaque position du long peptide synthétique afin de produire une ou plusieurs valeurs de caractéristiques de clivage, en utilisant un modèle entraîné avec un vecteur de probabilité de clivage en entrée et une ou plusieurs valeurs de caractéristiques de clivage en sortie ;
une unité de sortie d'affinité de liaison qui reçoit en entrée des séquences peptidiques de néoantigènes correspondant à une séquence HLA de classe I et à une séquence HLA de classe II au sein des peptides longs synthétiques afin de produire une ou plusieurs valeurs de caractéristiques de néoantigènes liées à l'immunité et à l'affinité de liaison présentes dans le peptide long synthétique, en utilisant un modèle entraîné avec une séquence peptidique de néoantigène, une séquence HLA de classe I et une séquence HLA de classe II en entrées et une ou plusieurs valeurs de caractéristiques de néoantigènes en sortie ; et
une unité de sortie de score d'immunogénicité qui génère un score d'immunogénicité du long peptide synthétique, en utilisant un modèle entraîné avec la ou les valeurs de caractéristiques d'antigène, la ou les valeurs de caractéristiques de clivage et la ou les valeurs de caractéristiques de néoantigène en tant qu'entrées et un score d'immunogénicité en tant que sortie.

10. Dispositif de prédiction de l'immunogénicité selon la revendication 9, dans lequel :
le vecteur de probabilité de clivage pour chaque position du long peptide synthétique est un vecteur de probabilité de clivage pour chaque position, lorsqu'un long peptide synthétique est clivé par des protéosomes ou des cathepsines, qui sont des enzymes de clivage présentes chez le sujet.

11. Dispositif de prédiction de l'immunogénicité selon la revendication 9, dans lequel :
l'unité de sortie d'affinité de liaison
fournit une première valeur de caractéristique de néoantigène liée à l'immunité en utilisant des données d'activité des lymphocytes T et un modèle appris avec l'immunité aux peptides néoantigènes,
fournit une deuxième valeur de caractéristique de néoantigène correspondant à la liaison du peptide néoantigène présent dans le long peptide synthétique à l'aide du modèle appris en entrant les données de liaison pour le peptide néoantigène et les classes HLA I et II, et
fournit une troisième valeur de caractéristique de néoantigène qui est le produit de la première valeur de caractéristique de néoantigène et de la deuxième valeur de caractéristique de néoantigène.

12. Dispositif de prédiction de l'immunogénicité selon la revendication 9, dans lequel :
l'unité de sortie de caractéristiques d'antigène
fournit une première valeur de caractéristique antigénique à l'aide d'un modèle appris en intégrant les informations de séquence du long peptide synthétique,
fournit une deuxième valeur de caractéristique d'antigène à l'aide d'un modèle appris à partir de données obtenues par codage one-hot des informations de séquence du long peptide synthétique, et
fournit une troisième valeur de caractéristique antigénique à l'aide d'un modèle appris à partir de données obtenues par traitement BLOSUM des informations de séquence du long peptide synthétique.

13. Dispositif de prédiction de l'immunogénicité selon la revendication 9, dans lequel :
le peptide néoantigénique a une longueur de 40 mères ou moins.

14. Dispositif de prédiction de l'immunogénicité selon la revendication 9, dans lequel :
le long peptide synthétique est formé de telle sorte qu'une séquence peptidique néoantigénique correspondant à HLA de classe I soit positionnée au centre.
